(19) Europäisches Patentamt
European Patent Office
Office européen des brevets

(11) Veröffentlichungsnummer: **0 196 023**
**A2**

(12) **EUROPÄISCHE PATENTANMELDUNG**

(21) Anmeldenummer: **86103868.5**

(22) Anmeldetag: **21.03.86**

(51) Int. Cl.⁴: **C 07 C 113/04**, C 09 B 35/08, D 06 P 3/68

(30) Priorität: **29.03.85 DE 3511546**

(43) Veröffentlichungstag der Anmeldung: **01.10.86 Patentblatt 86/40**

(84) Benannte Vertragsstaaten: **BE CH DE FR GB IT LI NL**

(71) Anmelder: **HOECHST AKTIENGESELLSCHAFT, Postfach 80 03 20, D-6230 Frankfurt am Main 80 (DE)**

(72) Erfinder: **Hertel, Hasso, Dr., Brunnenweg 10, D-6052 Mühlheim am Main (DE)**
Erfinder: **Hunger, Klaus, Dr., Johann-Strauss-Strasse 35, D-6233 Kelkheim (Taunus) (DE)**
Erfinder: **Frölich, Heinrich, Dr., Obernhäuser Weg 6, D-6272 Niedernhausen/Taunus (DE)**

(54) **4,4'-Diazoverbindungen von 3,3'-Dialkoxy-biphenylen, Verfahren zu ihrer Herstellung und deren Verwendung.**

(57) bis-Diazoverbindungen der Formel (1)

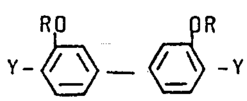

(1)

worin R einen geradkettigen oder verzweigten Alkyl- oder Alkoxyalkylrest von 3 bis 5 Kohlenstoffatomen bedeutet, und Y den anti-Diazotatrest $-N{=}N{-}O^{-}ME^{+}$, worin ME für ein Kalium- oder Natriumatom steht, oder den Rest $-N{=}N{-}Z$, worin Z für den Rest eines wasserlöslichen aliphatischen oder aromatischen Amins enthaltend eine Sulfonsäure- und/oder eine Carbonsäuregruppe steht, bedeutet, und Verfahren zu ihrer Herstellung, indem man ein Diamin der Formel (2)

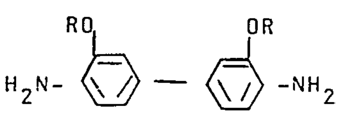

(2)

worin R die vorstehend genannten Bedeutungen hat, in einer wäßrigen, starken, nichtoxidierenden anorganischen oder organischen Säure mit einem Alkalimetallnitrit bei Temperaturen von etwa −10°C bis etwa +40°C bis-diazotiert und die gebildete bis-Diazoniumverbindung entweder in das anti-bis-Diazotat der genannten Formel (1) mit $Y = -N{=}N{-}O^{-}ME^{+}$ oder in eine bis-Diazoaminoverbindung der Formel (1) mit $Y = -N{=}N{-}Z$ in an sich bekannter Weise überführt und dann abscheidet.

EP 0 196 023 A2

4,4'-Diazoverbindungen von 3,3'-Dialkoxy-biphenylen, Verfahren zu ihrer Herstellung und deren Verwendung

Die vorliegende Erfindung betrifft neue 4,4'-Diazoverbindungen von 3,3'-Dialkoxy-biphenylen, Verfahren zu ihrer Herstellung und deren Verwendung zum Färben oder Bedrucken von Textilfasermaterialien nach den Methoden der Eisfarbentechnik, d.h. zur Erzeugung von wasserunlöslichen Azofarbstoffen, insbesondere auf der Faser, unter Verwendung geeigneter Kupplungskomponenten (siehe beispielsweise Hermann Rath, Lehrbuch der Textilchemie, 2. Auflage, Seiten 367-372,Springer-Verlag Berlin/Göttingen/Heidelberg 1963), sowie zur Herstellung von Lichtpausen nach den Methoden der Reprografie (vgl. Kirk-Othmer, Encyclopedia of Chemical Technology, Second Edition, Volume 17, Seiten 358-364, Interscience Publishers, New York, 1968).

Gefunden wurden neue 4,4'-Diazoverbindungen der allgemeinen Formel (1)

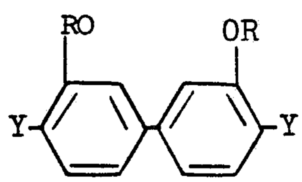

(1)

in welcher R einen geradkettigen oder verzweigten Alkyl- oder Alkoxyalkylrest von insgesamt 3 bis 5 Kohlenstoffatomen bedeutet, und Y als Diazorest für den anti-Diazotatrest $-N{=}N{-}O^{-}Me^{+}$, worin Me für ein Kalium- oder Natriumatom steht, oder den Rest -N=N-Z, worin Z für den Rest eines wasserlöslichen aliphatischen oder aromatischen Amins enthaltend eine Sulfonsäure- und/oder eine Carbonsäuregruppe, vorzugsweise die Gruppierung $-N(CH_3)-CH_2-CH_2-SO_3Me$, $-N(CH_3)-CH_2-COOMe$ oder -N(Me)-CN steht, wobei Me ein Kalium- oder Natriumatom bedeutet, Verfahren zu ihrer Herstellung, indem man ein Diamin der allgemeinen Formel (2)

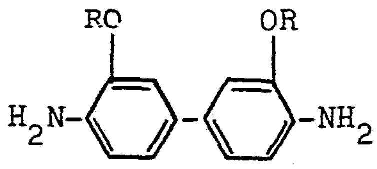 (2)

in welcher R die vorstehend genannten Bedeutungen hat, in einer wäßrigen, starken, nichtoxidierenden anorganischen oder organischen Säure, wie Schwefelsäure, Phosphorsäure, Chloressigsäure oder vorzugsweise Salzsäure, mit einem Alkalimetallnitrit, vorzugsweise Natriumnitrit, bei Temperaturen von etwa -10°C bis etwa +40°C, vorzugsweise 0 bis 30°C, bis-diazotiert und die gebildete bis-Diazoniumverbindung entweder in das anti-bis-Diazotat der genannten Formel (1) mit Y = $-N{=}N-O^{-}Me^{+}$ oder in eine bis-Diazoaminoverbindung der Formel (1) mit Y = -N=N-Z in an sich bekannter Weise überführt und dann abscheidet.

Die wäßrige, starke Säure, in welcher die Bis-diazotierung vorgenommen wird, hat zweckmäßigerweise eine Konzentration von etwa 5 % bis etwa 18 %. Die im Einzelfall angewendete optimale Konzentration richtet sich nach der eingesetzten Säure. Es ist zweckmäßig, pro Mol Diamin 4,4 bis 10 Äquivalente, vorzugsweise 5 bis 6 Äquivalente an Säure anzuwenden.

Als Diamine der genannten Formel (2) kommen in Betracht 4,4'-Diamino-3,3'-dipropoxy-biphenyl, 4,4'-Diamino-3,3'-bis-(1-methylethoxy)-biphenyl, 4,4'-Diamino-3,3'-dibutoxy-biphenyl, 4,4'-Diamino-3,3'-bis-(1-methyl-propoxy)-biphenyl, 4,4'-Diamino-3,3'-bis-(2-methylpropoxy)-biphenyl, 4,4'-Diamino-3,3'-bis-(1,1-dimethyl-ethoxy)-biphenyl, 4,4'-Diamino-3,3'-dipentoxy-biphenyl, 4,4'-Diamino-3,3'-bis-(3-methylbutoxy)-biphenyl, 4,4'-Diamino-3,3'-bis-(2-methoxy-ethoxy)-biphenyl und 4,4'-Diamino-3,3'-bis-(2-ethoxy-ethoxy)-biphenyl.

Die Überführung der verfahrensgemäß zunächst in Lösung angefallenen Diazoniumverbindung in das entsprechende anti-Diazotat erfolgt in an sich bekannter Weise, indem man die zunächst hergestellte Diazoniumsalzlösung des Diamins der genannte Formel (2) in gekühlte oder heiße wäßrige Alkalimetallhydroxydlösung, beispielsweise etwa 40-50 %ige Kalilauge oder Natronlauge, bei Temperaturen von etwa 10-20°C einlaufen läßt, wobei zunächst eine Suspension des syn-Diazotats anfällt. Durch Erwärmen der Suspension erfolgt eine Umwandlung in das anti-Diazotat. Nach beendeter Umlagerung wird abgekühlt, zweckmäßigerweise auf 20-30°C, wobei das anti-Diazotat sich in kristalliner Form abscheidet. Es wird über ein alkaliresistentes Filter abgesaugt.

Es kann anschließend im Warmluftstrom getrocknet werden. Zweckmäßiger ist aber, das angefallene feuchte anti-Diazotat durch Zentrifugieren von der anhaftenden Mutterlauge weitgehend zu befreien und sodann in einem mechanischen Mischer, beispielsweise in einem Pflugschaufel- oder Doppelschnecken-Mischer, mit wasserfreiem Natriumacetat zu vermischen, wobei zweckmäßigerweise pro Teil Restfeuchte zwei Teile Natriumacetat (wasserfrei) eingesetzt werden.

Zur Überführung der zunächst hergestellten Diazoniumverbindung in die anti-Diazotatverbindung kann man statt von der gelösten Diazoniumverbindung auch von dem bereits isolierten Diazoniumsalz ausgehen.

Das auf diese Weise erhaltene Präparat (anti-Diazotat) kann zur Herstellung von Drucken auf Textilgeweben, vorzugsweise solchem aus nativen oder regenerierten Cellulosefasern, verwendet werden, wobei wie folgt gearbeitet wird:

Eine Präparatmenge mit einem Gehalt von beisielsweise 13,2 Teilen 100%igem bis-anti-Diazotat wird in beispielsweise 350 Teilen Wasser gelöst, die mit 5 Teilen 32%iger Natronlauge alkalisch eingestellt worden waren. Dazu gibt man eine Lösung einer geeigneten Kupplungsverbindung, die beispielsweise durch Einrühren in ein Gemisch aus Ethanol, 32%iger Natronlauge und Wasser (im Gewichtsverhältnis 10:7:20) bereitet wurde. Durch Zugabe von Verdickungsmittel und Wasser wird auf eine geeignete Druckpaste mit der notwendigen Viskosität eingestellt.

Die so hergestellte Druckpaste kann mittels einer Sieb- oder Rouleaux-Druckmaschine beispielsweise auf ein Baumwollgewebe gedruckt werden. Das bedruckte Gewebe wird anschließend getrocknet und in einem Dämpfer Wasserdampf, der Essigsäure enthalten kann ausgesetzt, wobei nach Rückbildung des kupplungsfähigen Diazoniumsalzes aus dem anti-Diazotat der entsprechende wasserunlösliche Azofarbstoff auf der Faser gebildet wird. Zur Fertigstellung des Drucks wird wie üblich nachbehandelt.

Die Überführung der verfahrensgemäß zunächst in Lösung angefallenen Diazoniumverbindung in die entsprechende Diazoaminoverbindung erfolgt in an sich bekannter Weise, indem man die zunächst erhaltene Diazoniumsalzlösung zu einer wäßrig- alkalischen Lösung eines aliphatischen sekundären Amins oder aromatischen Amins, wie beispielsweise der Methylamino-essigsäure, 2-Methylamino-ethansulfonsäure oder Sulfanthranilsäure gibt, wobei das Medium bei einem pH-Wert von mindestens 9, vorzugsweise 10-11 gehalten wird. Anschließend wird die gebildete bis-Diazoaminoverbindung, beispielsweise durch Zugabe von Natriumchlorid, ausgesalzen, anschließend abfiltriert, durch Zentrifugieren von der

Hauptmenge der anhaftenden Mutterlauge befreit und schließlich bei mäßiger Temperatur, beispielsweise im Luftstrom bei 40°C getrocknet.

Zur Umwandlung der zunächst hergestellten Diazoniumverbindung in die Diazoaminoverbindung kann man statt von der gelösten Diazoniumverbindung auch von dem bereits isolierten Diazoniumsalz ausgehen.

Die so hergestellten und isolierten Diazoaminoverbindungen können zur Herstellung von Drucken auf Textilgeweben, vorzugsweise von solchem aus nativen oder regenerierten Cellulosefasern, eingesetzt werden, indem man wie folgt arbeitet:

Die Diazoaminoverbindung wird in einer mittels Natronlauge alkalisch gestellten Wassermenge gelöst. Zu dieser alkalischen Lösung gibt man eine Lösung einer geeigneten Kupplungskomponente, die durch Einrühren der Kupplungskomponente in ein Gemisch aus beispielsweise Ethanol, Natronlauge und Wasser (im Mengenverhältnis 10:7:20) bereitet wurde. Durch Zugabe von Verdickung und Wasser wird auf eine geeignete Druckpaste mit der erforderlichen Viskosität eingestellt.

Die so hergestellte Druckpaste kann mittels einer Sieb- oder Rouleaux-Druckmaschine beispielsweise auf Baumwollgewebe gedruckt werden. Das bedruckte Gewebe wird anschließend getrocknet und in einem Dämpfer Wasserdampf, der Essigsäure enthalten kann, ausgesetzt, wobei die Diazoaminoverbindung zur kupplungsfähigen Diazoniumverbindung gespalten und der entsprechende wasserunlösliche Azofarbstoff auf der Faser gebildet wird.

Beispiel 1

328 Teile 4,4'-Diamino-3,3'-dibutoxy-biphenyl werden in 600 Teile Wasser eingetragen und darin gut verrührt. Dann werden 260 Teile 32%iger Salzsäure zugetropft, wobei sich ein Brei des Hydrochlorids bildet. Nach einstündigem Nachrühren zur Vervollständigung der Hydrochloridbildung werden weitere 320 Teile 32%iger Salzsäure zugegeben. Durch Außenkühlung wird auf ca. 20°C abgekühlt. Dann läßt man 370 Teile einer 40%igen wäßrigen Lösung von Natriumnitrit so rasch zutropfen, daß Nitrit zunächst nicht oder nur schwach im Überschuß ist. Die Temperatur steigt dabei auf 30-35°C an und wird bis zum Ende der Diazotierung dort gehalten. Nach beendeter Diazotierung soll ein deutlicher Nitritüberschuß vorhanden sein.

Anschließend gibt man 10 Teile Klärkohle zu. Nach etwa 5 Minuten wird durch Tüpfeln der Auslauf geprüft. Ist er rein gelb, so wird nach Zugabe von 10 Teilen Kieselgur abgesaugt, ist er noch braun, so muß erneut Kohle zugegeben werden. Der Rückstand wird mit wenig Wasser gewaschen.

Die so hergestellte und geklärte Diazolösung wird in 2000 Teile 48%iger Kalilauge eingetragen. Die Temperatur wird dabei bei 10-20°C gehalten. Die ersten Anteile werden langsam zugegeben, weil zunächst eine schmierige Fällung entsteht, die aber bald pulvrig zerfällt. Von da an kann das Eintragen so rasch erfolgen, wie es die Kühlung erlaubt. Es resultiert eine dünne, hellgelbe Suspension des syn-Diazotats.

Zur Umlagerung in das anti-Diazotat wird erwärmt. Es tritt eine Farbveränderung nach braun ein. Der Fortgang der Reaktion wird durch Lösen einer Probe in etwas Wasser und Tüpfeln mit alkalischer 2-Hydroxy-naphthalin-3-carbonsäure-phenylamid-Lösung verfolgt. Tritt keine Blaufärbung mehr auf, so ist die Umlagerung beendet. Der zwischenzeitlich etwas dick gewordene Ansatz ist in diesem Stadium wieder dünn. Das Produkt hat sich zu einer weichen Masse zusammengeballt und die Temperatur hat ca. 100°C erreicht.

Nach beendeter Umlagerung wird sogleich durch Außenkühlung wieder auf 20-30°C abgekühlt. Das erhaltene anti-Diazotat der Formel

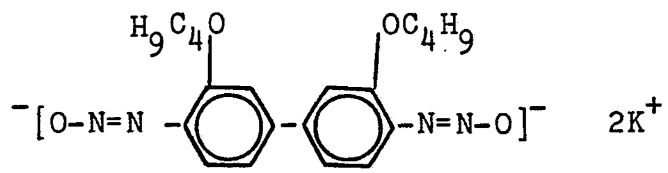

zerfällt dabei zu einem braunen Pulver, das über ein alkali-resistentes Filter abgesaugt wird.

Das Pulver kann im Warmluftstrom bei 40°C getrocknet werden. Zweckmäßiger wird es aber durch Zentrifugieren von der anhaftenden Mutterlauge weitgehend befreit und sodann in einem mechanischen Mischer, z.B. einem Pflugschaufel- oder einem Doppelschnecken-Mischer, mit wasserfreiem Natriumacetat vermischt, wobei pro Teil Restfeuchte zwei Teile Natriumacetat verwendet werden.

Das hierbei erhaltene Präparat kann zur Herstellung von Drucken auf Geweben aus nativen oder regenerierten Cellulosefasern verwendet werden, wobei wie folgt gearbeitet wird:

Eine Produktmenge mit einem Gehalt von 13,2 Teilen 100%igem bis-anti-Diazotat der angegebenen Formel wird in ca. 350

Teilen Wasser gelöst, die mit 5 Teilen 32%iger Natronlauge alkalisch gemacht worden waren. Dazu gibt man eine Lösung von 12,5 Teilen 2-Hydroxy-naphthalin-3-carbonsäure-phenylamid, die durch Einrühren in ein Gemisch aus 10 Teilen Ethanol, 7 Teilen 32 %iger Natronlauge und 20 Teilen Wasser bereitet wurde. Mit Druckverdickung und Wasser wird auf 1000 Teile verdünnt und dabei die notwendige Viskosität eingestellt.

Die so erhaltene Druckpaste druckt man auf einer Sieb- oder Rouleaux-Druckmaschine auf Baumwollgewebe, trocknet es und führt es sodann durch einen Dämpfer, in dem es essigsaurem Wasserdampf ausgesetzt ist. Dabei entsteht ein dunkelblaues Druckmuster, welches zur Fertigstellung wie üblich nachbehandelt wird.

Beispiel 2

Eine nach Beispiel 1 hergestellte Diazolösung läßt man bei ca. 10°C in eine Vorlage aus 244 Teilen Methylaminoessigsäure-Natrium in 1400 Teilen Wasser zulaufen. Durch Zugabe von Natronlauge wird dabei ein pH-Wert von 10 gehalten. Die Reaktionslösung ist zunächst rotbraun, hellt sich aber nach beendeter Zugabe oder auch bei einer Unterbrechung des Zulaufs nach braungelb auf. Nach Einstreuen von 500 Teilen Natriumchlorid scheidet sich die bis-Diazoaminoverbindung der Formel

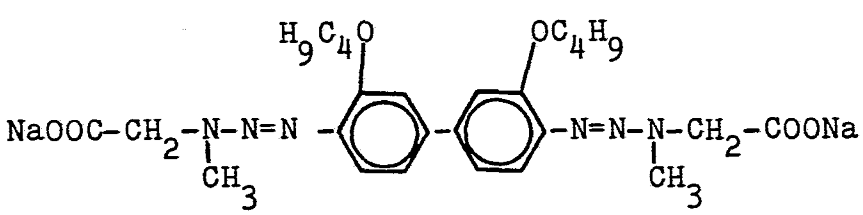

in feinen gelben Kristallen ab. Sie wird abgesaugt, dann durch Zentrifugieren von der Hauptmenge der anhaftenden Mutterlauge befreit und schließlich im Warmluftstrom von 40°C getrocknet.

Beispiel 3

Ersetzt man in Beispiel 2 die 244 Teile Methylaminoessigsäure-Natrium durch 355 Teile 2-Methylamino-ethansulfonsäure-Natrium, so erhält man die Diazoaminoverbindung der Formel

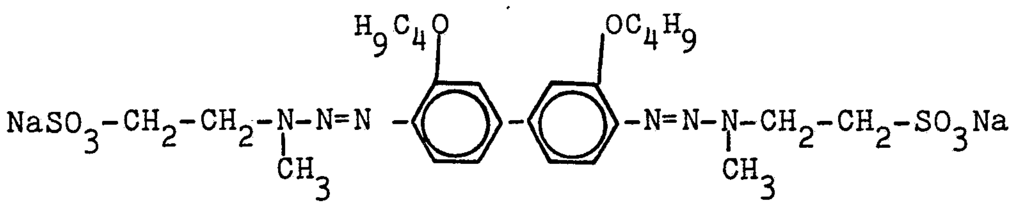

in ebenfalls feinen, gelben Kristallen.

Mit den gemäß Beispielen 2 und 3 erhaltenen Produkten kann man Drucke auf Geweben aus nativen oder regenerierten Cellulosefasern auf folgende Weise erzeugen:

Ein nach Beispiel 2 hergestelltes Produkt mit einem Gehalt von 15,3 Teilen 100%igem bis-Triazen der dort angegebenen Formel wird in ca. 350 Teilen Wasser gelöst, die mit 5 Teilen 32%iger Natronlauge alkalisch eingestellt worden waren. Dazu gibt man eine Lösung von 12,5 Teilen 2-Hydroxy-naphthalin-3carbonsäure-phenylamid, die durch Einrühren in ein Gemisch aus 10 Teilen Ethanol, 7 Teilen 32 %iger Natronlauge und 20 Teilen Wasser bereitet worden war. Durch Zugabe von Verdickungsmittel und Wasser wird so auf 1000 Teile eingestellt, daß dabei die notwendige Viskosität erzielt wird.

Diese Druckpaste druckt man auf einer Sieb- oder Rouleaux-Druckmaschine auf Baumwollgewebe. Nach Trocknen des bedruckten Gewebes wird dieses durch einen Dämpfer, in dem es essigsaurem Wasserdampf ausgesetzt ist, geführt. Dabei entsteht ein dunkelblaues Druckmuster. Zur Fertigstellung wird wie üblich nachbehandelt.

Man erhält ein identisches Druckmuster, wenn man statt des nach Beispiel 2 erhaltenen Präparats ein nach Beispiel 3 hergestelltes Präparat mit einem Gehalt von 19,2 Teilen dort genannten 100%igen bis-Triazens einsetzt.

Beispiel 4

300 Teile 4,4'-Diamino-3,3'-dipropoxy-biphenyl werden mit 550 Teilen Wasser verrührt. Innerhalb einer Stunde läßt man 580 Teile 32%ige Salzsäure zutropfen. Nach dem Abkühlen auf etwa 10°C läßt man eine 40%ige wäßrige Lösung von 140 Teilen Natriumnitrit in einem solchen Tempo zulaufen, daß die Reaktion auf freie salpetrige Säure negativ oder nur schwach positiv ist. Die Temperatur wird durch Außenkühlung auf 10-15°C gehalten.

Nach beendeter Diazotierung ist ein schwacher, aber deutlicher Nitritüberschuß vorhanden. Klärkohle und Filterhilfsmittel werden zugegeben. Nach einigen Minuten wird filtriert. Der Rückstand wird mit wenig Wasser gewaschen.

Die vereinigten Filtrate werden unter Kühlung (T = 10-20°C) in 1800 Teile 48%ige Kalilauge eingetragen, wobei man zu Beginn langsam arbeitet, bis eine kristalline Fällung erfolgt ist. Zu der entstandenen Suspension des syn-Diazotats werden 1000 Teile festes Kaliumhydroxyd gegeben. Dann wird zur Umlagerung in das anti-Diazotat erwärmt. Wenn die Umlagerung beendet ist (Nachweis durch Tüpfelreaktion, vgl.

Beispiel 1) wird auf ca. 30°C abgekühlt. Dabei kann durch Zugabe von Wasser die Kalilaugekonzentration bis auf ca. 25 % abgesenkt werden, um eine bessere Filtration zu ermöglichen.

Das ausgeschiedene anti-Diazotat der Formel

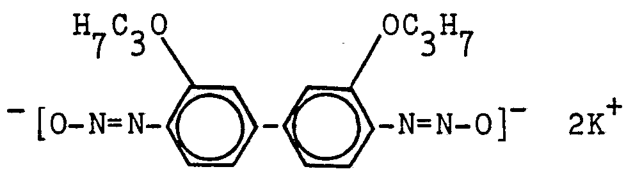

wird über ein alkali-resistentes Filter abgesaugt.

Es kann entweder im Warmluftstrom von 40°C oder durch Vermischen mit wasserfreiem Natriumacetat getrocknet werden. Im letzteren Fall wird die anhaftende Feuchte vom zugemischten Natriumacetat als Hydrat gebunden.

Beispiel 5

300 Teile 4,4'-Diamino-3,3'-di-iso-propoxy-biphenyl werden mit 600 Teilen Wasser verrührt. Dazu läßt man 580 Teile 32%ige Salzsäure zulaufen. Dann wird auf 0°C abgekühlt und durch Zugabe von 370 Teilen einer 40%igen wäßrigen Natriumnitritlösung diazotiert, wobei die Temperatur bei 0-10°C gehalten wird. Nach beendeter Diazotierung wird nach Zugabe von Klärkohle und Filterhilfsmittel filtriert.

Das Filtrat wird langsam zu 2200 Teilen 48%iger heißer Kalilauge (T ca. 100°C) gegeben. Wenn kein syn-Diazotat mehr nachweisbar ist, wird auf ca. 20°C abgekühlt. Das ausgefallene anti-Diazotat der Formel

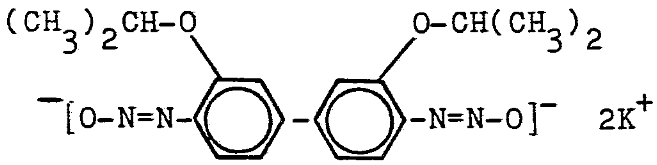

wird abgesaugt und nach einer der in Beispiel 4 angegebenen Methoden getrocknet.

Beispiel 6

Arbeitet man wie in Beispiel 5 angegeben, verwendet aber anstelle von Kalilauge Natronlauge, so erhält man ein anti-Diazotat der Formel

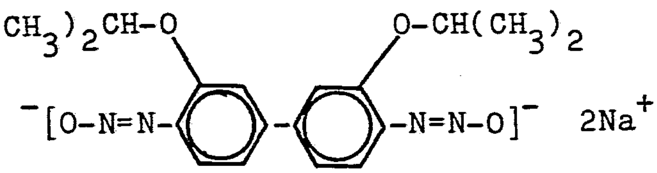

Beispiel 7

300 Teile 4,4'Diamino-3,3'-dipropoxy-biphenyl werden, wie in Beispiel 4 angegeben, in eine Diazolösung überführt. Diese läßt man bei ca. 10°C in eine Vorlage aus 244 Teilen Methylaminoessigsäure-Natrium und 1400 Teilen Wasser laufen, wobei der pH-Wert durch Zugabe von Natronlauge bei 10 gehalten wird.

Nach beendeter Zugabe werden 400 Teile Natriumchlorid eingestreut, wobei sich die bis-Diazoaminoverbindung der Formel

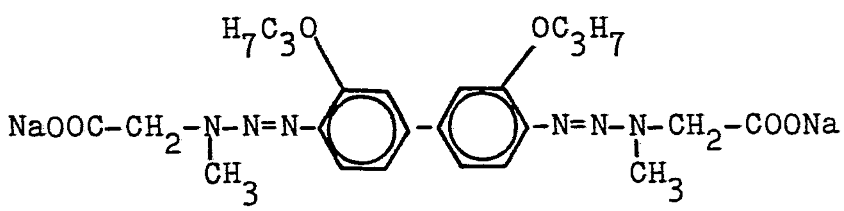

abscheidet. Sie wird abgesaugt und im Warmluftstrom von 40°C getrocknet.

Beispiel 8

300 Teile 4,4'-Diamino-3,3'-di-iso-propoxy-biphenyl werden, wie in Beispiel 5 angegeben, in eine Diazolösung überführt. Diese wird bei ca. 10°C zu einer Lösung von 355 Teilen 2-Methylamino-ethansulfonsäure-Narium in 1800 Teile Wasser gegeben, wobei der pH-Wert durch Zugabe von Natronlauge bei 10 gehalten wird.

Nach Zugabe von 500 Teilen Natriumchlorid wird die bis-Diazoaminoverbindung der Formel

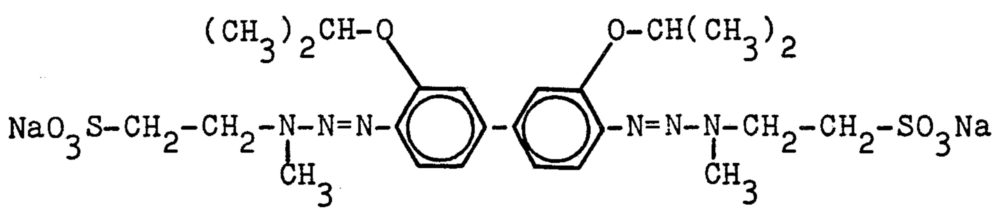

abgesaugt und Warmluftstrom von 40°C getrocknet.

Beispiel 9

332 Teile 4,4'-Diamino-3,3'-(2-methoxy-ethoxy)-biphenyl werden mit 550 Teilen Wasser, 580 Teilen 32%iger Salzsäure und 370 Teilen 40%iger Natriumnitritlösung nach dem Verfahren des Beispiels 1 diazotiert.

Die geklärte Diazolösung läuft bei ca. 10°C in eine Lösung von 355 Teilen 2-Methylamino-ethansulfonsäure-Natrium in 1600 Teilen Wasser, wobei durch Zugabe von Natronlauge ein pH-Wert von 10 gehalten wird.

Nach Zugabe von 500 Teilen Natriumchlorid wird die ausgeschiedene bis-Diazoaminoverbindung der Formel

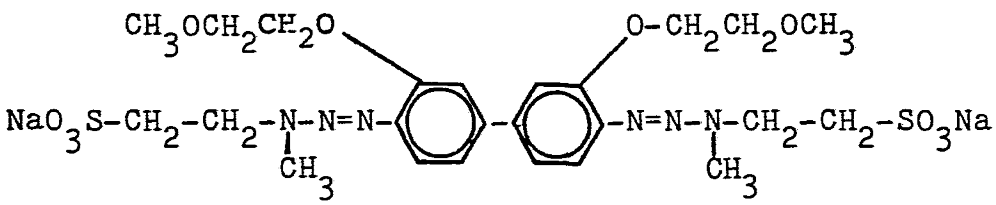

abgesaugt und Warmluftstrom von 40°C getrocknet.

Beispiel 10

328 Teile 4,4'-Diamino-3,3'-di-butoxy-biphenyl werden, wie in Beispiel 1 angegeben, diazotiert.

Die geklärte Diazolösung läuft bei 0-10°C in eine Lösung von 120 Teilen Cyanamid in 6000 Teilen Wasser, wobei durch Zugabe von Natronlauge ein pH-Wert von 10 gehalten wird. Nach beendeter Zugabe wird durch Einleiten von Dampf die Temperatur auf 50°C erhöht und sodann der pH-Wert mit Natronlauge auf 12 gestellt. Dann wird auf 25°C abgekühlt, das ausgeschiedene bis-Triazen nach Zugabe von 100 Teilen Natriumchlorid abgesaugt und zur Entfernung von anhaftender Mutterlauge zentrifugiert.

Getrocknet wird durch Vermischen mit wasserfreiem Natriumacetat.

PATENTANSPRÜCHE

1. bis-Diazoverbindungen der allgemeinen Formel

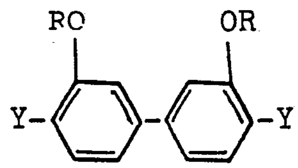

in welcher R einen geradkettigen oder verzweigten Alkyl- oder Alkoxyalkylrest von insgesamt 3 bis 5 Kohlenstoffatomen bedeutet, und Y den anti-Diazotatrest $-N=N-O^{-}Me^{+}$, worin Me für ein Kalium- oder Natriumatom steht, oder den Rest -N=N-Z, worin Z für den Rest eines wasserlöslichen aliphatischen oder aromatischen Amins enthaltend eine Sulfonsäure- und/oder eine Carbonsäuregruppe steht, bedeutet.

2. bis-Diazoverbindungen der in Anspruch 1 genannten allgemeinen Formel, in welcher Z im Rest -N=N-Z die Gruppierung $-N(CH_3)-CH_2-CH_2-SO_3Me$, $-N(CH_3)-CH_2-COOMe$ oder -N(Me)-CN bedeutet, in welchen Me für ein Kalium- oder Natriumatom steht.

3. bis-Diazoverbindungen der in Anspruch 1 genannten allgemeinen Formel, in welcher R eine n-Propyl-, iso-Propyl-, n-Butyl-, iso-Butyl-, n-Pentyl-, iso-Pentyl-, 2-Methoxyethyl- oder 2-Ethoxy-ethyl-Gruppe bedeutet.

4. Verfahren zur Herstellung von bis-Diazoverbindungen der allgemeinen Formel (1)

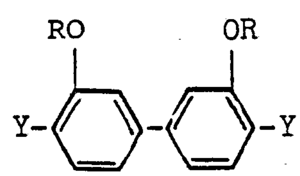

(1)

in welcher R einen geradkettigen oder verzweigten Alkyl- oder Alkoxyalkylrest von insgesamt 3 bis 5 Kohlenstoffatomen bedeutet, und Y den anti-Diazotatrest $-N=N-O^{-}Me^{+}$, worin Me für ein Kalium- oder Natriumatom steht, oder den Rest -N=N-Z, worin Z für den Rest eines wasserlöslichen aliphatischen oder aromatischen Amins enthaltend eine Sulfonsäure- und/oder eine Carbonsäuregruppe, steht, bedeutet, dadurch gekennzeichnet, daß man ein Diamin der allgemeinen Formel (2)

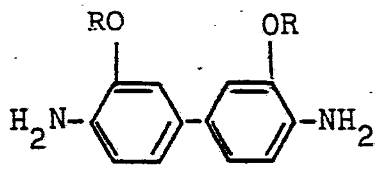

(2)

in welcher R die vorstehend genannten Bedeutungen hat, in einer wäßrigen, starken, nichtoxidierenden anorganischen oder organischen Säure mit einem Alkalimetallnitrit bei Temperaturen von etwa -10°C bis etwa +40°C bis-diazotiert und die gebildete bis-Diazoniumverbindung entweder in das anti-bis-Diazotat der genannten Formel (1) mit Y = $-N=N-O^{-}Me^{+}$ oder in eine bis-Diazoaminoverbindung der Formel (1) mit Y = -N=N-Z in an sich bekannter Weise überführt und dann abscheidet.

5. Verfahren nach Anspruch 4, dadurch gekennzeichnet, daß man die Diazotierung in wäßriger Salzsäure, Schwefelsäure, Phosphorsäure oder Monochloressigsäure bei 0 bis 30°C durchführt.

6. Verfahren nach Anspruch 4, dadurch gekennzeichnet, daß man die Überführung der bis-Diazoniumverbindung in das anti-bis-Diazotat durch Zugabe der Diazoniumsalzlösung in heiße Kali- oder Natronlauge oder in kalte Kali- oder Natronlauge mit anschließendem Erwärmen der Mischung vornimmt.

7. Verfahren nach Anspruch 4, dadurch gekennzeichnet, daß man die Überführung der bis-Diazoniumverbindung in die bis-Diazoaminoverbindung durch Zugabe der Diazoniumsalzlösung zu einer wäßrig-alkalischen Lösung eines aliphatischen sekundären Amins oder aromatischen Amins, jeweils enthaltend eine Sulfonsäure- und/oder eine Carbonsäuregruppe, wobei der pH-Wert des Mediums mindestens 9 ist.

8. Verwendung der bis-Diazoverbindungen nach Anspruch 1 zum Färben oder Bedrucken von Fasermaterialien aus nativer oder regenerierter Cellulose.

9. Verwendung der bis-Diazoverbindungen nach Anspruch 1 zur Herstellung von Lichtpausen nach den Methoden der Reprografie.